# EUROPEAN PATENT SPECIFICATION

(11) **EP 2 549 978 B1**
(45) Date of publication and mention of the grant of the patent: **16.07.2014**
(21) Application number: 11808067.0
(22) Date of filing: 16.12.2011
(51) Int. Cl.: A61K 8/42, A61K 8/49, A61K 8/67, A61Q 5/00, A61Q 7/00, A61Q 5/06, A61Q 5/12

(54) **HAIR CARE COMPOSITIONS AND METHODS TO IMPROVE HAIR APPEARANCE**
HAARPFLEGEZUSAMMENSETZUNGEN UND VERFAHREN ZUR VERBESSERUNG DES HAARAUSSEHENS.
COMPOSITIONS DE SOINS CAPILLAIRES ET PROCÉDÉS POUR AMÉLIORER L'ASPECT DES CHEVEUX

(30) Priority: 21.12.2010 US 201061425659 P
(43) Date of publication of application: 30.01.2013
(73) Proprietor: The Procter & Gamble Company, Cincinnati, OH 45202 (US)
(72) Inventor: DAWSON, Thomas, Larry, Jr., Hamilton Ohio 45011 (US); YOUNGQUIST, Robert, Scott, Mason Ohio 45040 (US); RICHARDS, Jeanette, Anthea, Liberty Township Ohio 45044 (US); XIE, Sancai, West Chester Ohio 45069 (US); COMBS, Mary, Jane, Covington Kentucky 41011 (US)
(74) Representative: Holmes, Rosalind
(86) International application number: PCT/US2011/065345
(87) International publication number: WO 2012/087779

(56) References cited:
- WO-A1-2009/107073
- WO-A2-2008/027541
- WO-A2-2009/135006
- US-A1- 2006 067 905

## Description

### FIELD OF THE INVENTION

The present invention relates to hair care compositions and methods that provide for healthy and younger looking hair which can increase the appearance of thicker and/or fuller hair, longer hair, and/or delay the appearance of gray hair.

### BACKGROUND OF THE INVENTION

Many attributes contribute to the appearance of hair considered to be attractive. For instance, hair with a full and thick appearance is very desirable. In contrast, hair with a thin appearance is not as attractive, and can even lead to a perception that the thin-haired individual is older than their chronological age. Additionally, the appearance of gray hair can also lead to the perception that an individual is older than their chronological age. Furthermore, thin hair and gray hair can be more difficult to style, and typically cannot be styled into as many hairstyles, leaving the individual frustrated and with an unkempt appearance. Because of the foregoing problems associated with thin hair and graying hair, many individuals expend great effort and time on grooming, yet still do not attain their desired hairstyle and appearance. This can lead to frustration and/or lack of confidence in his or her appearance. These problems can be experienced by both female and male consumers and at a variety of ages.

Accordingly, there is a need to provide consumers with a way to increase the fullness and thickness of hair appearance and reduce the appearance of gray hair, thus resulting in healthier and younger-looking, more attractive hair appearance.

### SUMMARY OF THE INVENTION

The present invention relates to hair care compositions and methods that can help increase the appearance of fuller and/or thicker hair and/or reduce the appearance of gray hair, thus resulting in healthier and younger-looking hair. This result is achieved by increasing the diameter of hair shafts and follicles, increasing the number of hairs, reducing the emergence of gray hairs, growing longer hair, and/or having hair with less damage. Claimed is a hair care composition comprising:
a. from 0.15% to 3% apigenin
b. from 0.1 % to 10% caffeine
c. from 0.1% to 25% niacinamide, and
d. from 0.01% to 3% panthenol.

Also claimed is the use of the hair care composition as claimed (see above for providing younger looking hair by providing a benefit selected from the group consisting of increasing the hair shaft diameter, increasing hair density, increasing the length of anagen phase, longer hair, delayed emergence of gray hair, and combination thereof.- Also claimed is a method comprising applying the composition according to a regimen, wherein said regimen comprises:
(a) cleansing the scalp and/or face to form a cleansed scalp and/or face;
(b) topically applying the composition to said cleansed scalp and/or cleansed face;
wherein the composition is a composition as claimed (see above).

In one aspect, a method comprises topically applying a hair care composition comprising an effective amount of a hair growth inhibiting agent to the scalp for the purpose of improving the appearance of the hair. In a particular embodiment, the hair growth inhibiting agent comprises a material that up-regulates aquaporin-3 expression.

In a further embodiment, the hair care composition comprises a synergistic mixture of apigenin, a xanthine compound, a vitamin B3 compound, and a panthenol compound. In a particular embodiment, the synergistic mixture comprises apigenin, caffeine, niacinamide, and panthenol. In one embodiment, the composition comprises from 0.15% to 3% of apigenin, in another embodiment from 0.2% to 2%, and in yet another embodiment from 0.5% to 1.5%. In some embodiment, the composition comprises from 0.1 % to 10% of a xanthine compound (e.g., caffeine), in another embodiment from 0.5% to 5% of a xanthine compound, and in yet another embodiment from 1% to 2% of a xanthine compound. In some embodiments, the composition comprises from 0.1% to 25% of a vitamin B3 compound (e.g., niacinamide), in another embodiment from 0.5% to 15% of a vitamin B3 compound, and in yet another embodiment from 3.5% to 7.5% of a vitamin B3 compound. In some embodiments, the composition comprises from 0.01% to 3% of a panthenol compound (e.g., panthenol), in another embodiment from 0.02% to 1% of a panthenol compound, and in yet another embodiment from 0.2% to 0.5% of a panthenol compound. In one embodiment, the composition also comprises a thickener that helps to hold the active agents on the scalp, providing substantivity to the composition, such that it does not drip undesirably onto unintended areas of the body, clothing, or home furnishings.

These and other features, aspects, and advantages of the present invention will become evident to those skilled in the art from a reading of the present disclosure.

### DETAILED DESCRIPTION OF THE INVENTION

While the specification concludes with the claims particularly pointing and distinctly claiming the invention, it is believed that the present invention will be better understood from the following description. All percentages, parts and ratios are based upon the total weight of the hair care compositions of the present invention and all measurements made are at 25°C, unless otherwise specified. All such weights as they pertain to listed ingredients are based on the active level and, therefore, do not include carriers or by-products that may be included in commercially available materials, unless otherwise specified.

As used herein, the term "hair care compositions" are compositions that are applied to the hair and/or the skin underneath the hair, including compositions used to treat or care for the hair. Products contemplated by the phrase "hair care composition" include, but are not limited to liquids, creams, wipes, hair conditioners (rinse-off and leave-on), hair tonics, shampoos, hair colorants, mousses, propellant lotions, foams, emulsions, shave gels, after-shave tonics and lotions, temporary beard hair dyes, and the like.

"Hair growth inhibiting agent" or "hair growth inhibition agent" includes any material that can reduce, inhibit, attenuate, or diminish mammalian hair growth.

"Increase the appearance of fuller and thicker hair" means the diameters of hair follicles and/or shafts in the subject region of hair (e.g., scalp, beard) are increased by a statistically significant amount, when an effective amount of a composition of the present invention is topically applied to the desired region over a result-effective period of time.

"Delay the appearance of gray hair" means the rate of gray hair emerging is delayed. This can be measured by visual observation and by the method described in Japanese patent application 2005-296352A assigned to Shiseido. The counting method consists of designating a 50 mm x 10 mm area on either side of the frontal scalp and collecting all the hairs within the area and counting 1000 hairs cut from the area. Gray hairs and pigmented hairs are both counted. The process is repeated monthly, or as desired, and the percent of gray hairs is calculated.

"Mammalian hair," as referenced herein, includes hair on any part of the body of a mammal, and can include but is not limited to facial, cranial, or body hair. For instance, it can include hair on the scalp, head, neck, beard, moustache, eyebrows and sideburns hair.

The term "topical application," as used herein, means to apply or spread the compositions of the present invention onto the surface of the keratinous tissue from which the hair to be affected grows.

The term "dermatologically-acceptable," as used herein, means that the compositions or components thereof so described are suitable for use in contact with mammalian keratinous tissue without undue toxicity, incompatibility, instability, allergic response, and the like.

The term "effective amount," as used herein, means an amount of a compound or composition sufficient to increase the diameter of the shafts in the subject region of hair by a statistically significant amount, to increase the hair density (number of hairs per area) by a statistically significant amount, and/or to delay the appearance of gray hair by a statistically significant amount.

Unless otherwise specified, in all embodiments of the present invention, all percentages are by weight of the total composition, unless specifically stated otherwise. All ratios are weight ratios, unless specifically stated otherwise. All ranges are inclusive and combinable. The number of significant digits conveys neither limitation on the indicated amounts nor on the accuracy of the measurements. All numerical amounts are understood to be modified by the word "about" unless otherwise specifically indicated. All measurements are understood to be made at 25°C and at ambient conditions, where "ambient conditions" means conditions under about one atmosphere of pressure and at about 50% relative humidity.

In the past, various compositions comprising hair growth inhibition agents have been applied to body areas where hair is not desired, in an effort to eliminate, decrease, and/or slow the growth of unwanted hair. Contrary to this conventional wisdom, applicants have found that a combination of topical application of hair growth inhibitors to regions where the appearance of more hair is desired can actually increase the appearance the health of the hair by having an appearance of thicker and/or fuller hair and/or delay the appearance of gray hair. For example, previous articles have taught that apigenin has been useful for regulating mammalian hair growth, including retarding or inhibiting or eliminating hair growth (See US 2008/0254055A1 and 6,239,170).

Although not wishing to be limited by theory, it is believed that topical application of various hair growth inhibitors stimulate aquaporin 3 ("AQP3") up-regulators, which in turn results in thicker hair shafts and follicles. The topical application may also help to slow the rate in which hair leaves the anagen phase and delay the appearance of gray hair. The increase in the diameter of the hair shafts and follicles leads to the appearance of hair that is thicker and fuller. Furthermore, the topical application can lead to the appearance of younger looking hair, since hair diameter is known to decrease with one's chronological age and the appearance of gray hair can be delayed.

Aquaporins ("AQP") are a family of water-channel proteins found in the plasma membrane. Currently, 13 AQP have been identified in humans, and they are classified into two groups: aquaporins and aquaglyceroporins. Most of the identified aquaporins belong to the aquaporin group that is selective primarily for the passage of water. AQP 3, 7, 9, and 10 belong to the aquaglyceroporin group that facilitates the movement of water, glycerol, and various other solutes. Through immunocytochemistry, applicants have found that AQP3 is strongly expressed in the proliferating keratinocytes of the hair follicles. In addition, applicants have also found that AQP3 immunostaining can be detected in the hair shaft. It is believed by applicants that AQP3 up-regulators increase the thickness of hair fibers and follicles by stimulating AQP3 expression, which allows more water and water-binding molecules to be transported into the cells, thus improving cellular metabolism and increasing cellular size. This leads to increased hydration of the hair's cuticle layer, which expands the cuticle, making it thicker. As a result, the diameter of the hair fiber and of the hair follicle increases, resulting in the appearance of thicker, fuller hair.

A composition comprising a combination of hair growth inhibitors that stimulate aquaporin 3 up-regulators is provided. A synergistic combination of materials of the present invention is desired. A combination of apigenin and caffeine may aid in up-regulation and help with younger looking hair.

The topical application of a hair care composition of present invention can aid in lengthening the anagen phase. The lengthening of the anagen phase can be achieved by either blocking the transition from anagen phase to telogen phase or by inhibiting the transition from anagen phase to telogen phase. The hair follicles are in a growing phase (anagen) or in a resting phase (telogen). Follicles are predominately in the anagen phase. The anagen phase typically lasts for approximately 2 to 10 years. This length will vary. Conversely, the telogen phase is much shorter and is typically for approximately 3 months. In general, a person will have approximately 94% of the follicles in anagen phase and 6% of the follicles in telogen phase. Each month approximately 2% of the follicles leave anagen phase and transition to telogen phase and at the same time approximately 2% of the follicles leave telogen phase and transition to anagen phase. With the application of the hair care compositions of the present invention, the approximately 2% of the follicles leaving anagen phase can be either blocked or delayed resulting in an increased percent of hair follicles in anagen phase. The increase in the amount of follicles in anagen phase increases the hair density on the head. It is believed that the length of the anagen phase can be increased from about 2 weeks to about 2.5 months. The increase in hair density (number of hair on a certain area of the scalp) can be measured. In one embodiment of the present invention, the hair density will increase by 2 hairs/cm2, preferably by 3 hairs/cm2, and in some embodiments greater than 4 hairs/cm2. The benefits of the increased anagen phase, hair density, and hair diameter and the delay of gray appearance will result in a person's hair looking from 3 or more years younger.

The use of a combination of hair growth inhibiting agents has the purpose of increasing the appearance of younger looking hair.

### A. Hair Care Compositions

Claimed is a hair care composition comprising: (a) from 0.15% to 3% apigenin, (b) from 0.1% to 10% caffeine, (c) from 0.1% to 25% niacinamide, and (d) from 0.01% to 3% panthenol. In one aspect, the present invention provides hair care compositions that can be used to increase the appearance of thicker and fuller hair. The hair care composition comprises a combination of hair growth inhibiting agents. Preferably, the hair growth inhibiting agent is present in a safe and effective amount. Optionally, the hair care compositions can comprise a dermatologically-acceptable carrier and/or any desired suitable optional ingredients.

The further hair growth inhibition agents can be selected from the group consisting of butylated hydroxytoluene, butylated hydroxyanisole, hexamidine, hexyl isobutyrate, menthyl anthranilate, methofuran, 3-butylidenepthalide, glyceryl dilaurate, hexanediol, agmatine, aminoguanidine, phenyl butyl nitrone and other spin traps, ethoxyquin, cetyl pyridinium chloride, green tea extract, catechins, phytosterols, ursolic acid, apigenin, plant extracts, plant extract compounds, 3-butylidenepthalide, its salts, its derivatives, and mixtures thereof and combinations thereof. In one embodiment, the composition comprises apigenin, a xanthine compound, a vitamin B3 compound, a panthenol compound, or mixtures thereof. Particular materials are described in more detail below.

### 1. Apigenin

Claimed is a hair care composition comprising: (a) from 0.15% to 3% apigenin. The composition of the present invention includes the flavonoid, apigenin (4',5,7-trihydroxyflavone). Apigenin, 5,7-Dihydroxy-2-(4-hydroxyphenyl)-4H-1-benzopyran-4-one , is a nonmutagenic citrus bioflavonoid which is present in many type of plants and vegetables. Examples include, but are not limited to, grapefruit, parsley, thyme, chamomile, apples, celery, basil, oregano, tarragon, cilantro, yarrow, , and passion flower. Although apigenin can be found in many of the plants and vegetables, for the present invention, the apigenin must be at least 95% pure and more preferably at least 98% pure. The total concentration of apigenin in these materials varies greatly and there may not be enough apigenin present in the materials to be useful for the present invention. For the present invention, the apigenin itself must be present in an amount of at least 0.15% based on the total composition. Claimed is a hair care composition comprising: (a) from 0.15% to 3% apigenin. The apigenin may be present in amount of greater than 0.2%, 0.25%, 0.3%, 0.4%, 0.5%, 0.7%, 0.9, and 1%. The apigenin is typically present in an amount of less than 3%, 2.5%, 2%, 1.8%, 1.6%, 1.5%, 1.4%, 1.3%, 1.2%, 1%, 0.9%, 0.8%, 0.7%, 0.6%, 0.5%, 0.4%, 0.3%, and 0.2%.

### 2. Xanthine Compounds

The compositions of the present invention include a xanthine compound (caffeine). Claimed is a hair care composition comprising: (b) from 0.1% to 10% caffeine. As used herein, "xanthine compound" means one or more xanthines, derivatives thereof, and mixtures thereof. Xanthine compounds that can be useful herein include, but are not limited to, caffeine, xanthine, 1-methylxanthine, theophylline, theobromine, derivatives thereof, and mixtures thereof. The composition comprises from 0.1% to 10% of a xanthine compound (caffeine). In another embodiment the composition comprises from 0.5% to 5% of a xanthine compound, and in yet another embodiment from 1% to 2% of a xanthine compound.

### 3. Vitamin B₃ Compounds

The compositions of the present invention include a vitamin B3 compound (niacinamide). Claimed is a hair care composition comprising: (c) from 0.1% to 25% niacinamide. Vitamin B3 compounds are particularly useful for regulating skin conditions, as described in U.S. Patent No. 5,939,082. The composition comprises from 0.1% to 25% of a vitamin B3 compound (niacinamide). In another embodiment the composition comprises from 0.5% to 15% of a vitamin B3 compound, and in yet another embodiment from 3.5% to 7.5% of a vitamin B3 compound. As used herein, "vitamin B3 compound" means one or more compounds having the formula: 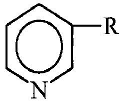 wherein R is - CONH₂ (i.e., niacinamide), - COOH (i.e., nicotinic acid) or - CH2OH (i.e., nicotinyl alcohol); derivatives thereof; mixtures thereof; and salts of any of the foregoing.

Exemplary derivatives of the foregoing vitamin B3 compounds include nicotinic acid esters, including non-vasodilating esters of nicotinic acid (e.g, tocopherol nicotinate, myristyl nicotinate), nicotinyl amino acids, nicotinyl alcohol esters of carboxylic acids, nicotinic acid N-oxide and niacinamide N-oxide.

Suitable esters of nicotinic acid include nicotinic acid esters of C₁-C₂₂, preferably C₁-C₁₆, more preferably C₁-C₆ alcohols. The alcohols are suitably straight-chain or branched chain, cyclic or acyclic, saturated or unsaturated (including aromatic), and substituted or unsubstituted. The esters are preferably non-vasodilating. As used herein, "non-vasodilating" means that the ester does not commonly yield a visible flushing response after application to the skin in the subject compositions (the majority of the general population would not experience a visible flushing response, although such compounds may cause vasodilation not visible to the naked eye, i.e., the ester is non-rubifacient). Non-vasodilating esters of nicotinic acid include tocopherol nicotinate and inositol hexanicotinate; tocopherol nicotinate is preferred.

Other derivatives of the vitamin B₃ compound are derivatives of niacinamide resulting from substitution of one or more of the amide group hydrogens. Nonlimiting examples of derivatives of niacinamide useful herein include nicotinyl amino acids, derived, for example, from the reaction of an activated nicotinic acid compound (e.g., nicotinic acid azide or nicotinyl chloride) with an amino acid, and nicotinyl alcohol esters of organic carboxylic acids (e.g., C1 - C18). Specific examples of such derivatives include nicotinuric acid (C8H8N2O3) and nicotinyl hydroxamic acid (C6H6N2O2), which have the following chemical structures:
nicotinuric acid: 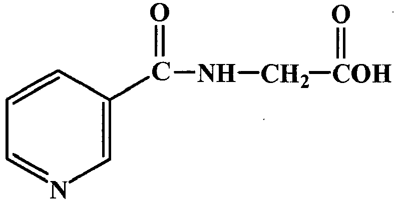
nicotinyl hydroxamic acid:

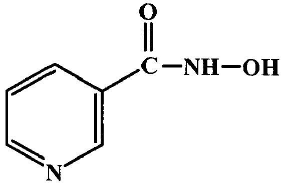

Exemplary nicotinyl alcohol esters include nicotinyl alcohol esters of the carboxylic acids salicylic acid, acetic acid, glycolic acid, palmitic acid and the like. Other non-limiting examples of vitamin B3 compounds useful herein are 2-chloronicotinamide, 6-aminonicotinamide, 6-methylnicotinamide, N-methylnicotinamide, N,N--diethylnicotinamide, N-(hydroxymethyl)-nicotinamide, quinolinic acid imide, nicotinanilide, n-benzylnicotinamide, N-ethylnicotinamide, nifenazone, nicotinaldehyde, isonicotinic acid, methyl isonicotinic acid, thionicotinamide, nialamide, 1-(3-pyridylmethyl) urea, 2-mercaptonicotinic acid, nicomol, and niaprazine.

Examples of the above vitamin B3 compounds are well known in the art and are commercially available from a number of sources, e.g., the Sigma Chemical Company (St. Louis, MO); ICN Biomedicals, Inc. (Irvine, CA) and Aldrich Chemical Company (Milwaukee, WI).

One or more vitamin B3 compounds may be used herein. Preferred vitamin B3 compounds are niacinamide and tocopherol nicotinate. Niacinamide is more preferred and claimed.

When used, salts, derivatives, and salt derivatives of niacinamide are preferably those having substantially the same efficacy as niacinamide.

Salts of the vitamin B3 compound are also useful herein. Nonlimiting examples of salts of the vitamin B3 compound useful herein include organic or inorganic salts, such as inorganic salts with anionic inorganic species (e.g., chloride, bromide, iodide, carbonate, preferably chloride), and organic carboxylic acid salts (including mono-, di- and tri- C1 - C18 carboxylic acid salts, e.g., acetate, salicylate, glycolate, lactate, malate, citrate, preferably monocarboxylic acid salts such as acetate). These and other salts of the vitamin B3 compound can be readily prepared by the skilled artisan, for example, as described by W. Wenner, "The Reaction of L-Ascorbic and D-Iosascorbic Acid with Nicotinic Acid and Its Amide", J. Organic Chemistry, Vol. 14, 22-26 (1949). Wenner describes the synthesis of the ascorbic acid salt of niacinamide.

In a preferred embodiment, the ring nitrogen of the vitamin B3 compound is substantially chemically free (e.g., unbound and/or unhindered), or after delivery to the skin becomes substantially chemically free ("chemically free" is hereinafter alternatively referred to as "uncomplexed"). More preferably, the vitamin B3 compound is essentially uncomplexed. Therefore, if the composition contains the vitamin B3 compound in a salt or otherwise complexed form, such complex is preferably substantially reversible, more preferably essentially reversible, upon delivery of the composition to the skin. For example, such complex should be substantially reversible at a pH of from 5.0 to 6.0. Such reversibility can be readily determined by one having ordinary skill in the art.

More preferably the vitamin B3 compound is substantially uncomplexed in the composition prior to delivery to the keratinous tissue. Exemplary approaches to minimizing or preventing the formation of undesirable complexes include omission of materials which form substantially irreversible or other complexes with the vitamin B3 compound, pH adjustment, ionic strength adjustment, the use of surfactants, and formulating wherein the vitamin B3 compound and materials which complex therewith are in different phases. Such approaches are well within the level of ordinary skill in the art.

Thus, in a preferred embodiment, the vitamin B3 compound contains a limited amount of the salt form and is more preferably substantially free of salts of a vitamin B3 compound. Preferably the vitamin B3 compound contains less than 50% of such salt, and is more preferably essentially free of the salt form. The vitamin B3 compound in the compositions hereof having a pH of from 4 to 7 typically contain less than 50% of the salt form.

The vitamin B3 compound may be included as the substantially pure material, or as an extract obtained by suitable physical and/or chemical isolation from natural (e.g., plant) sources. The vitamin B3 compound is preferably substantially pure, more preferably essentially pure.

### 4. Panthenol Compounds

The compositions of the present invention comprise a panthenol compound (panthenol). Claimed is a hair care composition comprising: (d) from 0.01% to 3% panthenol. As used herein, the term "panthenol compound" is broad enough to include panthenol, one or more pantothenic acid derivatives, and mixtures thereof. Panthenol and its derivatives can include D-panthenol ([R]-2,4-dihydroxy-N-[3-hydroxypropyl)]-3,3-dimethylbutamide), DL-panthenol, pantothenic acids and their salts, preferably the calcium salt, panthenyl triacetate, royal jelly, panthetine, pantotheine, panthenyl ethyl ether, pangamic acid, pantoyl lactose, Vitamin B complex, or mixtures thereof.

Compositions comprising pantothenic acid derivatives that remain more stable than panthenol and other similar materials in acidic compositions or in compositions containing acid-producing materials such as aluminum-containing actives, can also be suitable for use herein. The selected pantothenic acid derivatives are most typically in liquid form and dispersed throughout or otherwise solubilized within the liquid carrier component of the composition.

The term "pantothenic acid derivative" as used herein refers to those materials that conform to the formula: 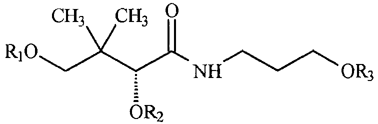 wherein R₁, R₂ and R₃ are hydrogen, C2-C20 hydrocarbons, C2-C20 carboxylic acid esters, or combinations thereof, provided that not more than two of R1, R2 and R3 are hydrogen. In one embodiment, R₁, R₂ and R₃ are independently selected from hydrogen, C2-C8 hydrocarbons, C2-C8 carboxylic acid esters, or combinations thereof; in another embodiment, R₁ and R₂ are hydrogen, and R₃ is a C2-C8 hydrocarbon, C2-C8 carboxylic acid ester, or combinations thereof; in yet another embodiment, R₁ and R₂ are hydrogen and R₃ is ethyl. The selected pantothenic acid derivatives may be derived or otherwise obtained from any known source, which may include pantothenic acid or materials other than pantothenic acid, so long as the resulting material has the above defined chemical formula.

Specific non-limiting examples of selected pantothenic acid derivatives for use herein include ethyl panthenol, panthenyl triacetate, and combinations thereof. In a particular embodiment, a pantothenic acid derivative comprises the d-isomeric form(s) of such derivative form(s), such as d-ethyl panthenol. Claimed is a hair care composition comprising: (d) from 0.01% to 3% panthenol. In one embodiment, the composition will contain from 0.01% to 5% of a panthenol compound. Typically, compositions will contain from 0.03% to 3%, from 0.05% to 2%, and from 0.1% to 1%, by weight of the final composition.

### 5. Optional Ingredients

The compositions of the present invention can also additionally comprise any suitable optional ingredients as desired. For example, the composition can optionally include other active or inactive ingredients.

For instance, the present invention may include additional skin care actives selected from the group consisting of sugar amines, retinoids, peptides, dialkanoyl hydroxyproline, hexamidine, salicylic acid, phytosterol, sunscreen actives, water soluble vitamins, oil-soluble vitamins, their derivatives, their precursors, and combinations thereof. Furthermore, the composition may include suitable ingredients that are conventionally used in given product types. The compositions may also include other common hair ingredients such as pyrithione zinc, minoxidil, silicones, conditioning agents, and other suitable materials. The CTFA Cosmetic Ingredient Handbook, Tenth Edition (published by the Cosmetic, Toiletry, and Fragrance Association, Inc., Washington, D.C.) (2004) (hereinafter "CTFA"), describes a wide variety of nonlimiting materials that can be added to the composition herein. Examples of these ingredient classes include, but are not limited to: abrasives, absorbents, aesthetic components such as fragrances, pigments, colorings/colorants, essential oils, skin sensates, astringents, etc. (e.g., clove oil, menthol, camphor, eucalyptus oil, eugenol, menthyl lactate, witch hazel distillate), anti-acne agents, anti-caking agents, antifoaming agents, antimicrobial agents (e.g., iodopropyl butylcarbamate), antioxidants, binders, biological additives, buffering agents, bulking agents, chelating agents, chemical additives, colorants, cosmetic astringents, cosmetic biocides, denaturants, drug astringents, external analgesics, film formers or materials, e.g., polymers, for aiding the film-forming properties and substantivity of the composition (e.g., copolymer of eicosene and vinyl pyrrolidone), opacifying agents, pH adjusters, propellants, reducing agents, sequestrants, skin bleaching and lightening agents, (e.g. hydroquinone, kojic acid, ascorbic acid, magnesiuim ascorbyl phosphate, ascorbyl glucoside, pyridoxine), skin-conditioning agents (e.g. humectants and occlusive agents), skin treating agents (e.g. vitamin D compounds, mono-,di-, and tri-terpenoids, beta-ionol, cedrol), thickeners, hair conditioning agents, and surfactants.

In one embodiment, the composition comprises a thickener to increase the substantivity of the composition, such that it does not drip undesirably onto other areas of the body, onto clothing, or onto home furnishings. Any suitable thickener can be used, for example, a cellulose-based thickener such as hydroxypropylmethylcellulose. In some embodiments, the composition comprises alcohol, dipropylene glycol, and/or water.

The formulations of the present invention may be present in typical hair care compositions. They may be in the form of solutions, dispersion, emulsions, powders, talcs, encapsulated, spheres, spongers, solid dosage forms, foams, and other delivery mechanisms. The composition of the present invention may be hair tonics, leave-on hair products such as conditioners, treatment, and styling products, rinse-off hair products such as conditions, shampoos, and treatment products; and any other form that may be applied to the hair and preferably applied to the scalp.

The hair care composition of the present invention will contain apigenin. The apigenin may be in the same phase as the xanthine, vitamin B3, and panthenol or it may be in a separate phase or, not claimed herein, in a separate product. If two products are used, the products may be used together and at the same time or sequentially. Sequential use may occur in a short period of time, such as immediately after the use of one product, or it may occur over a period of hours or days.

### C. METHOD FOR INCREASING THE APPEARANCE OF THICKER AND FULLER HAIR AND/OR THE APPEARANCE OF GRAY HAIR

The present invention also provides a method for increasing the diameter of the hair shaft and follicle and increasing the density of hair follicles. This may lead to an appearance of thicker and/or fuller hair and may lead to the appearance of delayed onset of gray hair. In one aspect, the method comprises applying a hair care composition comprising a combination of hair growth inhibiting agents to a skin surface from which a region of hair grows. For instance, the hair care composition can be applied to the scalp. In another embodiment, the method comprises topically applying a hair care composition comprising an effective amount of a combination of hair growth inhibiting agents to a region of skin of a mammal seeking to increase the appearance of thicker and/or fuller hair or delaying the appearance of gray hair.

In still another embodiment, the method comprises applying the composition according to a regimen, wherein said regimen comprises:
(a) cleansing the scalp and/or face to form a cleansed scalp and/or face;
(b) topically applying the composition to said cleansed scalp and/or cleansed face.

The hair care composition of the present invention can be used daily, weekly, or in a variety of regimens. The hair care composition may be used more than once a day such as at night and in the morning. The product may be used after washing the hair which may mean use more than once per day on certain days or use only a few times per week. The hair care composition may be used three times per day, twice per day, once per day, six times per week, five times per week, four times per week, three times per week, two times per week, or one time per week. In some embodiments, the hair care composition is used four, five, six or seven times per week.

The hair care composition may be used by males and females. The composition may be desired to be used by individual who desired to promote hair growth or have healthier or younger looking hair. It has been found that the formulations of the present invention work surprising well in subjects who have little or no greater than normal hair loss. The formulation may be desired to be used upon subjects who have no diagnosed hair loss. The formulation can be used on subjects having an age of greater than 20, 25, 30, 35, 40, 45, or 50. The formulations can be used on subjects having an age of less than 70, 65, 60, 55, or 50. Formulations may be preferred for subjects between the ages of 20-70, from 30-60, and from 35-55. Hair diameter may start to decrease after age 20 so healthier hair and increased appearance of fuller and thicker hair may be desired after these ages. Hair diameter continues to decrease and in some subject to a greater extent after age 30 or 40. Additionally, gray hair begins to emerge as early as age 20 but more commonly after age 30 or 40 depending upon genetics.

### FORMULATIONS AND EXAMPLES

The following are non-limiting examples of the present invention.

In the examples, all concentrations are listed as weight percent, unless otherwise specified and may exclude minor materials such as diluents, filler, and so forth. The listed formulations, therefore, comprise the listed components and any minor materials associated with such components. As is apparent to one of ordinary skill in the art, the selection of these minors will vary depending on the physical and chemical characteristics of the particular ingredients selected to make the present invention as described herein.

### Example 1 and 2: Tonic example with synergistic combination

| | **1** | **2** |
|---|---|---|
| Apigenin | 1.00 | 1.00 |
| Dipropylene Glycol Low Odor grade (DPG LO) | 49.00 | 49.00 |
| Arlasolve DMI PC | 8.00 | 8.00 |
| Ethanol 40B | 18.60 | 8.60 |
| Hydrolite 5 | 10.00 | 10.00 |
| Tween 80 | 10.00 | 10.00 |
| Water | | 10.00 |
| Klucel H-CS | | |
| Dexpanthenol, USP | 0.15 | 0.15 |
| Caffeine | 0.75 | 0.75 |
| Niacinamide,USP | 2.50 | 2.50 |
| **Total** | 100.00 | 100.00 |

### Examples 3-16: Tonic with apigenin to be used in two step method, which is- not claimed

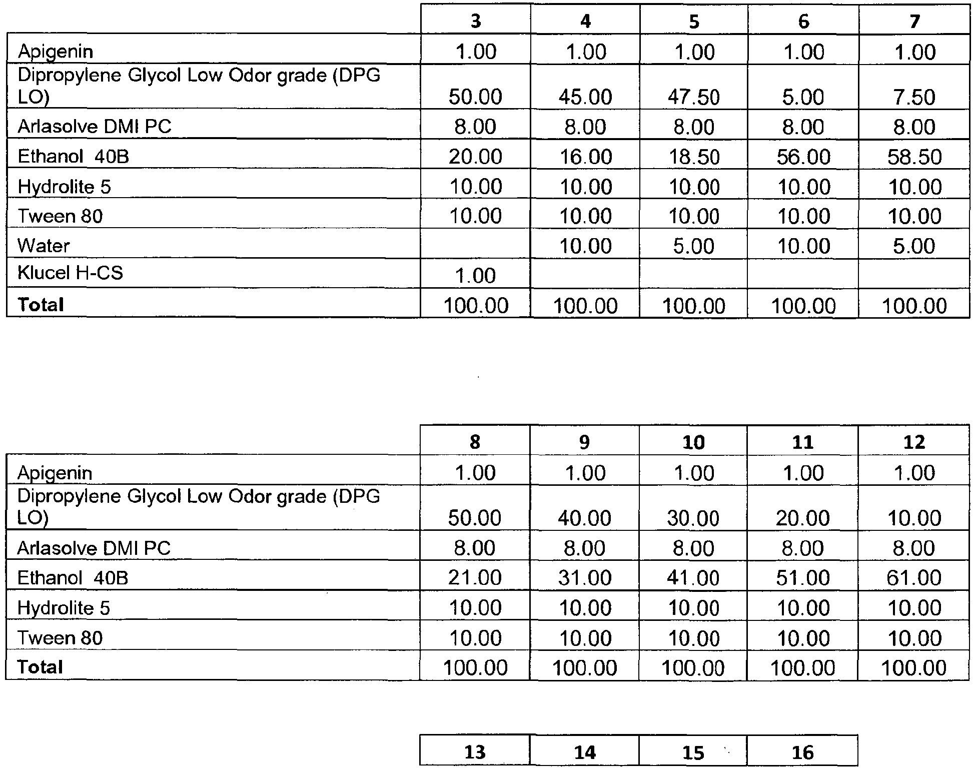

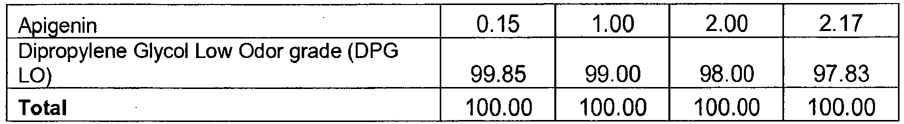

### Example 17: Tonic example for a two step method of treatment, which is not claimed

| | **17** |
|---|---|
| Alcohol 100% DEB 100 (Ethanol) | 25.00 |
| Carbomer (Carbopol Ultrez 10) | 0.10 |
| Hexamidine diisethionate | 0.10 |
| Glyceryl dilaurate | 2.00 |
| BHT | 0.50 |
| Triethanolamine | 0.20 |
| Caffeine | 1.50 |
| Niacinamide | 5.00 |
| Panthenol | 0.30 |
| Deionized water | Qs |

| **Raw Material** | **Supplier** |
|---|---|
| Apigenin | Sederma |
| Dipropylene Glycol Low Odor grade (DPG LO) | Lyondell Chemical Company |
| Arlasolve DMI PC | Croda |
| Ethanol 40B | Sasol |
| Hydrolite 5 | Symrise |
| Tween 80 | Ruger Chemical |
| Water | DI |
| Klucel H-CS | Hercules |
| Dexpanthenol, USP | DSM Nutritional Products |
| Caffeine | DSM Nutritional Products |
| Niacinamide,USP | Edison US |

### Data Examples

The dermal papilla, in normal human hair, is the control center for hair diameter and growth. Increasing survival of dermal papilla cells in situ leads to increased hair diameter and to a longer anagen phase which results in longer hair, a higher hair density, and potentially delayed emergence of gray hair. The appearance of this hair results in healthier, younger looking hair. Accordingly, an increase in survival of dermal papilla cells correlates with healthier hair showing an increase in hair diameter, longer hair, higher hair density, and a delayed on-set of gray hair.

An in vitro model, consisting of primary human dermal papilla cells in culture, demonstrates a surprising synergistic increase in the survival of metabolically stressed dermal papilla cells. Primary human dermal papilla cells were metabolically stressed for 48 hours in reduced standard tissue culture media. During this period of stress the cells were treated with the mixtures below. After 48 hr the metabolic activity of the cells was measured by the amount of ATP using the Cell Titer Glo™ kit (Promega).

The combination of apigenin plus caffeine, panthenol, and niacinamide provide surprisingly high results. The combination of caffeine, panthenol, and niacinamide provides 185% increase in the hair follicle cells that survived. The hair follicle cells treated with apigenin provided a 122% increase in the cell that survived. Surprising, the combination of apigenin with caffeine, panthenol, and niacinamide provided a 423% increase in the number of hair follicle cells that survived which is significantly higher than the predicted increase of the combination.

## Claims

1. A hair care composition comprising:
a. from 0.15% to 3% apigenin
b. from 0.1 % to 10% caffeine
c. from 0.1% to 25% niacinamide, and
d. from 0.01% to 3% panthenol

2. The composition according to claim 1, wherein the composition comprises from 0.2% to 2%, or from 0.5% to 1.5% apigenin.

3. The composition according to any of the preceding claims, wherein the composition also comprises a thickener, preferably a cellulose-based thickener such as hydroxypropylmethylcellulose.

4. The composition according to any of the preceding claims, wherein the composition also comprises a dermatologically-acceptable carrier.

5. The composition according to any of the preceding claims, wherein the composition includes additional skin care actives selected from the group consisting of sugar amines, retinoids, peptides, dialkanoyl hydroxyproline, hexamidine, salicylic acid, phytosterol, sunscreen actives, water soluble vitamins, oil-soluble vitamins, and combinations thereof

6. The composition according to any of the preceding claims, wherein the composition comprises alcohol, dipropylene glycol, and/or water.

7. The composition according to any of the preceding claims, wherein the apigenin was from one of grapefruit, parsley, thyme, chamomile, apples, celery, basil, oregano, tarragon, cilantro, yarrow, and passion flower.

8. The composition according to any of the preceding claims, in the form of a solution, dispersion, emulsion, powder, talc, encapsulate, sphere, sponger, solid dosage form, or foam.

9. The composition according to any of the preceding claims, wherein the composition is a hair tonic, leave-on hair product such as conditioners, treatment, and styling products, or rinse-off hair product such as conditioners, shampoos, and treatment products.

10. Use of the hair care composition of claim 1 for providing younger looking hair by providing a benefit selected from the group consisting of increasing the hair shaft diameter, increasing hair density, increasing the length of anagen phase, longer hair, delayed emergence of gray hair, and combination thereof

11. A method comprising applying the composition according to a regimen, wherein said regimen comprises:
(a) cleansing the scalp and/or face to form a cleansed scalp and/or face;
(b) topically applying the composition to said cleansed scalp and/or cleansed face;
wherein the composition is a composition according to any of claims 1 to 9.

12. A method for increasing hair shaft diameter, increasing hair density, and increasing the length of anagen phase to give the appearance of healthier hair comprising topically applying a hair care composition comprising a safe and effective amount of a hair care composition of claim 1 to a region where thicker and fuller hair is desired.

## Patentansprüche

1. Haarpflegezusammensetzung, umfassend:
a. 0,15 % bis 3 % Apigenin
b. 0,1 % bis 10 % Koffein
c. 0,1 % bis 25 % Niacinamid und
d. 0,01 % bis 3 % Panthenol

2. Zusammensetzung nach Anspruch 1, wobei die Zusammensetzung 0,2 % bis 2 % oder 0,5 % bis 1,5 % Apigenin umfasst.

3. Zusammensetzung nach einem der vorstehenden Ansprüche, wobei die Zusammensetzung auch ein Verdickungsmittel, vorzugsweise ein zellulosebasiertes Verdickungsmittel wie Hydroxypropylmethylcellulose umfasst.

4. Zusammensetzung nach einem der vorstehenden Ansprüche, wobei die Zusammensetzung auch einen hautverträglichen Träger umfasst.

5. Zusammensetzung nach einem der vorstehenden Ansprüche, wobei die Zusammensetzung zusätzliche Hautpflegewirkstoffe aufweist, die ausgewählt sind aus der Gruppe bestehend aus Zuckeraminen, Retinoiden, Peptiden, Di-alkanoylhydroxyprolin, Hexamidin, Salicylsäure, Phytosterol, Sonnenschutzwirkstoffen, wasserlöslichen Vitaminen, öllöslichen Vitaminen und Kombinationen davon.

6. Zusammensetzung nach einem der vorstehenden Ansprüche, wobei die Zusammensetzung Alkohol, Dipropylenglycol und/oder Wasser umfasst.

7. Zusammensetzung nach einem der vorstehenden Ansprüche, wobei das Apigenin von einer von Pampelmuse, Petersilie, Thymian, Kamille, Äpfeln, Sellerie, Basilikum, Oregano, Estragon, Koriander, Schafgarbe und Passionsblume stammt.

8. Zusammensetzung nach einem der vorstehenden Ansprüche, die in Form einer Lösung, Dispersion, Emulsion, eines Pulvers, Talks, Einkapselung, Kugel, Schwamms, fester Dosierungsform oder Schaums vorliegt.

9. Zusammensetzung nach einem der vorstehenden Ansprüche, wobei die Zusammensetzung ein Haartonikum, ein zum Belassen auf dem Haar bestimmtes Produkt wie Conditioner, Behandlungs- und Stylingprodukte oder ein zum Ausspülen aus dem Haar bestimmtes Produkt wie Conditioner, Shampoos und Behandlungsprodukte ist.

10. Verwendung der Haarpflegezusammensetzung nach Anspruch 1 zur Bereitstellung von jünger aussehendem Haar durch Bereitstellen einer Nutzwirkung, die ausgewählt ist aus der Gruppe bestehend aus dem Erhöhen des Haarschaftdurchmessers, Erhöhen der Haardichte, Verlängern der Anagenphase, längerem Haar, verzögertem Auftreten von grauem Haar und einer Kombination davon.

11. Verfahren, umfassend das Auftragen der Zusammensetzung gemäß einem System, wobei das System Folgendes umfasst:
(a) Reinigen der Kopfhaut und/oder Fläche zur Bildung einer gereinigten Kopfhaut und/oder Fläche,
(b) topisches Auftragen der Zusammensetzung auf die gereinigte Kopfhaut und/oder die gereinigte Fläche,
wobei die Zusammensetzung eine Zusammensetzung nach einem der Ansprüche 1 bis 9 ist.

12. Verfahren zum Erhöhen des Haarschaftdurchmessers, Erhöhen der Haardichte und Verlängern der Anagenphase, um dem Haar ein gesünderes Aussehen zu verleihen, umfassend das topische Auftragen einer Haarpflegezusammensetzung, die eine sichere und wirksame Menge einer Haarpflegezusammensetzung nach Anspruch 1 auf einen Bereich umfasst, an dem dickeres und volleres Haar gewünscht wird.

## Revendications

1. Composition pour soins capillaires comprenant :
a. de 0,15 % à 3 % d'apigénine
b. de 0,1 % à 10 % de caféine
c. de 0,1 % à 25 % de niacinamide, et
d. de 0,01 % à 3 % de panthénol

2. Composition selon la revendication 1, où la composition comprend de 0,2 % à 2 %, ou de 0,5 % à 1,5 % d'apigénine.

3. Composition selon l'une quelconque des revendications précédentes, où la composition comprend également un épaississant, de préférence un épaississant à base de cellulose tel que l'hydroxypropylméthylcellulose.

4. Composition selon l'une quelconque des revendications précédentes, où la composition comprend également un véhicule acceptable du point de vue dermatologique.

5. Composition selon l'une quelconque des revendications précédentes, où la composition inclut des principes actifs supplémentaires pour le soin de la peau, choisis dans le groupe constitué d'amines de sucre, rétinoïdes, peptides, dialcanoyl-hydroxyproline, hexamidine, acide salicylique, phytostérol, principes actifs contre les rayons solaires, vitamines hydrosolubles, vitamines oléosolubles, et leurs combinaisons.

6. Composition selon l'une quelconque des revendications précédentes, où la composition comprend de l'alcool, du dipropylène glycol, et/ou de l'eau.

7. Composition selon l'une quelconque des revendications précédentes, dans laquelle l'apigénine provenait d'un parmi le pamplemousse, le persil, le thym, la camomille, les pommes, le céleri, le basilic, l'origan, l'estragon, la coriandre, l'achillée mille-feuille, et la passiflore.

8. Composition selon l'une quelconque des revendications précédentes, sous la forme de solution, dispersion, émulsion, poudre, talc, gélule, sphère, éponge, forme pharmaceutique solide, ou mousse.

9. Composition selon l'une quelconque des revendications précédentes, où la composition est un tonique capillaire, un produit à laisser sur les cheveux tel que des conditionneurs, des produits de traitement et de coiffage, ou un produit capillaire à éliminer par rinçage tel que des conditionneurs, des shampooings, et des produits de traitement.

10. Utilisation de la composition pour soins capillaires selon la revendication 1 pour fournir des cheveux d'aspect plus jeunes en réalisant un effet bénéfique choisi dans le groupe constitué d'une augmentation du diamètre de la tige capillaire, une augmentation de la densité de cheveux, une augmentation de la longueur de phase anagène, des cheveux plus longs, une apparition retardée de cheveux gris, et une combinaison de ceux-ci.

11. Procédé comprenant l'application de la composition selon un schéma, dans lequel ledit schéma comprend :
(a) le nettoyage du cuir chevelu et/ou du visage pour former un cuir chevelu et/ou un visage nettoyés ;
(b) l'application locale de la composition auxdits cuir chevelu et/ou visage nettoyés ;
où la composition est une composition selon l'une quelconque des revendications 1 à 9.

12. Procédé pour augmenter le diamètre de la tige capillaire, augmenter la densité des cheveux, et augmenter la longueur de la phase anagène pour donner l'apparence de cheveux en meilleure santé comprenant l'application locale d'une composition pour soins capillaires comprenant une quantité sûre et efficace d'une composition pour soins capillaires selon la revendication 1 à une région où des cheveux épais et mieux remplis sont souhaités.
